(19) Europäisches Patentamt

European Patent Office

Office européen des brevets

(11) Numéro de publication: **0 309 353 B1**

(12) **FASCICULE DE BREVET EUROPEEN**

(45) Date de publication de fascicule du brevet: **27.07.94** (51) Int. Cl.⁵: **A61K 7/48**, A61K 9/12

(21) Numéro de dépôt: **88402390.4**

(22) Date de dépôt: **22.09.88**

(54) **Composition de traitement de la peau sous forme de mousse aérosol contenant de la vaseline.**

(30) Priorité: **23.09.87 US 100093**

(43) Date de publication de la demande:
**29.03.89 Bulletin  89/13**

(45) Mention de la délivrance du brevet:
**27.07.94 Bulletin  94/30**

(84) Etats contractants désignés:
**AT BE CH DE ES FR GB IT LI NL**

(56) Documents cités:
**EP-A- 0 103 911**
**EP-A- 0 262 681**
**FR-A- 2 137 684**

**G.A. NOWAK: "Die kosmetischen Präparate",
vol. 2, édition 3, Rezeptur,Rohstoffe, wissenschaftliche Grundlagen, 1984, pages
178-179,180-181, 886-887, 954-957, Verlag für
Chem. Industrie H. Ziolkowsky KG, Augsburg, DE; "Aerosol-Antiperspirant-Schaum";
p. 886, para. "O/W-Lichtschutzmilch"; p. 954,
para. "Sonnenschutz-Emulsion (O/W); p. 957,
para. "W/O-Emulsionen, versprühbar"; pp.
178-181, para. "HLB-Werte gebräuchlicher
Emulgatoren"**

(73) Titulaire: **L'OREAL**
**14, Rue Royale**
**F-75008 Paris(FR)**

(72) Inventeur: **Thau, Paul**
**181 Dogwood Lane**
**Berkeley Heights, N.J. 07922(US)**

(74) Mandataire: **Stalla-Bourdillon, Bernard et al**
**CABINET NONY & CIE**
**29, rue Cambacérès**
**F-75008 Paris (FR)**

Rank Xerox (UK) Business Services
(3. 10/3.09/3.3.3)

**Description**

La vaseline solide ou gel de petroleum, désigné ci-après par vaseline a été largement utilisée comme agent thérapeutique pour des applications locales. Cet agent thérapeutique possède des caractéristiques bien connues de lubrification, d'adoucissement et de traitement de la peau, et de nombreux dermatologues ont souscrit à l'utilisation de la vaseline comme agent efficace d'hydratation et de protection de la peau, pour des patients ayant une peau sèche ou squameuse.

La vaseline telle que définie ci-dessus ne doit pas être confondue avec l'huile de vaseline qui est un liquide à température ambiante qui est d'ailleurs couramment utilisée dans les domaines cosmétique et pharmaceutique et l'on peut citer, à cet égard, à titre d'exemple, la demande de brevet français n° 72.16568 (2.137.684) et la revue "Die Kosmetichen Präparate" (Nowak), vol.2, pp.178-181, 886-887 et 954-957.

La vaseline est constituée d'un mélange d'hydrocarbures (y compris de l'huile minérale et des cires d'hydrocarbure microcristallines) d'un type tel que lorsque le matériau fondu est refroidi à la température ambiante, il se congèle en une matière translucide amorphe ou du type gel.

La Pharmacopée U.S. (U.S.P.) emploie les termes de vaseline blanche et gel de petroleum blanc comme étant identiques et les décrit comme un mélange purifié d'hydrocarbures semi-solides obtenus à partir du pétrole et totalement décolorés ou presque. La vaseline est définie en outre comme ayant un domaine de point de fusion de 38°C à 60°C et comme ayant une consistance, telle que déterminée par la méthode officielle U.S.P., qui n'est pas inférieure à 100 et qui n'est pas supérieure à 275. Une qualité moins décolorée est décrite dans le National Formulary (N.F.) comme qualité "jaune" et comme exempte ou pratiquement exempte d'odeur et de saveur.

Parmi les nombreuses caractéristiques de la vaseline qui la rendent utile dans des applications topiques on peut mentionner son activité hydratante, sa propriété de barrière vis-à-vis de l'eau, son hydrophobie, sa résistance au lavage à l'eau, son inertie physiologique, et son inertie et sa stabilité chimiques.

Les principaux inconvénients de la vaseline dans les compositions par voie topique sont sa consistance grasse, son inélégance cosmétique et son manque d'aptitude à produire un film mince et consistant sur une grande surface de la peau.

Dans le passé, des compositions cosmétiques contenant de la vaseline ont été préparées en tentant d'éliminer les inconvénients associés à la vaseline par maintien d'une faible concentration, par exemple inférieure à 10 % en poids de la composition totale, ou par addition d'additifs pour réduire la sensation et l'aspect gras de la composition. Les compositions cosmétiques avec de faibles concentrations en vaseline ou contenant des additifs ne présentent pas souvent toutes les propriétés thérapeutiques de la vaseline. Le brevet U.S. n° 3 852 475, du 3 Décembre 1974, décrit un onguent contenant de la vaseline qui comprend un amidon hydrophobe destiné à réduire la sensation et l'aspect gras de l'onguent, ainsi que sa résistance au lavage. Toutefois, l'onguent du brevet U.S. n° 3 852 475 souffre cependant d'inélégance cosmétique et ne peut pas être facilement étendu sous forme d'un film mince sur une grande surface de la peau.

La présente invention élimine ces inconvénients associés à la vaseline dans des compositions cosmétiques en proposant une composition contenant de la vaseline sous forme d'une mousse aérosol. De plus, la mousse produite selon la présente invention a une densité de mousse pratiquement constante depuis la diffusion initiale jusqu'à la diffusion finale, contrairement aux mousses cosmétiques actuellement dans le commerce, dans lesquelles la densité de la mousse augmente de façon très importante lorsque le récipient se vide. De plus, la mousse selon la présente invention peut être produite à partir d'un concentrat ayant un rapport de concentration élevé de la vaseline à l'agent émulsionnant, maintenant ainsi l'efficacité d'hydratation de la peau par la vaseline.

La présente invention a pour objet une composition aérosol pour la formation d'une mousse, destinée à une application topique, comprenant une émulsion huile-dans-l'eau et un propulseur conditionnés sous pression dans un récipient aérosol, caractérisée par le fait que ladite émulsion huile-dans-l'eau comprend :

a) au moins 10 % en poids de vaseline, et généralement environ de 10 à environ 40 % en poids, et de préférence environ 12,5 à environ 35 % en poids,

b) au moins 50 % en poids d'eau et généralement environ de 50 à environ 80 % en poids et de préférence environ 55 à environ 77 % en poids, et

c) un agent émulsionnant ayant une balance hydrophile-lipophile de 6 à 10 et généralement présent en une proportion d'environ 2 à environ 10 % en poids, et de préférence environ 2 à environ 5 % en poids,

lesdits pourcentages étant exprimés par rapport au poids total de ladite émulsion huile-dans-l'eau,

et par le fait que ladite mousse a une densité de mousse qui varie de moins de 30 % entre la diffusion initiale et la diffusion finale de la composition à partir du récipient aérosol.

Dans un mode de réalisation de l'invention, il est proposé un conditionnement pour dispenser une mousse, qui comprend :

a) un récipient résistant à la pression ayant une ouverture commandée par une valve et une valve à commande manuelle ;

b) une émulsion huile-dans-l'eau utilisable comme concentrat aérosol produisant une mousse, contenu dans ledit récipient, et comprenant au moins 10 % en poids du poids total de l'émulsion de vaseline, au moins 50 % en poids d'eau et un agent émulsionnant ayant une balance hydrophile-lipophile d'environ 6 à 10, et

c) un propulseur contenu dans ledit récipient, qui est gazeux à la température ambiante et sous la pression atmosphérique.

De préférence, l'émulsion et le propulseur de la présente invention sont contenus dans un récipient résistant à la pression. Sous la forme la plus courante, le récipient est cylindrique avec un fond concave ou plat, et un dôme supérieur convexe avec une ouverture circulaire munie d'une valve. Le corps du récipient peut être en métal (comme le fer, l'aluminium ou l'étain), en verre ou en plastique. La valve est une valve standard utilisée dans l'industrie des aérosols pour produire une mousse. Le type ou la forme du récipient ne sont pas critiques pour la présente invention. En fonction de la présente description, il paraîtra évident pour les spécialistes des diffuseurs aérosols que l'on peut utiliser une grande variété de récipients dans la mise en oeuvre de l'invention.

L'émulsion de la présente invention est destinée à constituer un concentrat d'aérosol pour la production d'une mousse, contenu dans un récipient avec un propulseur, ladite émulsion étant une émulsion huile-dans-l'eau. Les émulsions huile-dans-l'eau produisent des mousses car le propulseur constitue la phase interne ; c'est-à-dire que le propulseur se vaporise sous la décharge et se dilate dans une matrice aqueuse continue pour former une mousse. L'émulsion comprend de la vaseline de l'eau et un agent émulsionnant. La vaseline peut être présente dans n'importe quelle proportion. De préférence, cependant, la proportion de vaseline est d'au moins 10 % en poids du poids total de l'émulsion. Dans les modes de réalisation préférés, la proportion de vaseline est d'environ 20 % en poids de l'émulsion. L'eau peut également être présente dans n'importe quelle proportion, mais il est préférable que l'émulsion contienne au moins 50 % en poids d'eau. On préfère en particulier les émulsions ayant une teneur en eau d'environ 55 à 77 % en poids.

Une grande variété d'agents émulsionnants peuvent être utilisés dans l'émulsion et peuvent consister en un seul agent émulsionnant ou plus couramment, en un mélange de plusieurs agents émulsionnants. Un agent émulsionnant est une molécule qui allie un groupe hydrophile (attiré par l'eau ou polaire) à un groupe lipophile (attiré par l'huile ou non polaire). Les agents émulsionnants sont souvent classés selon la balance entre la dimension et la force de leur groupe hydrophile et la dimension et la force de leur groupe lipophile. Cette balance est désignée comme la valeur de la balance hydrophile-lipophile (HLB) de l'agent émulsionnant. A un agent émulsionnant qui présente un caractère lipophile, on attribue un indice HLB faible (inférieur à environ 9) et à un agent émulsionnant qui présente un caractère hydrophile, on attribue un indice HLB élevé (supérieur à environ 11). Une discussion générale concernant le système de classification HLB des agents émulsionnants peut être trouvée dans "The HLB System : A Time-Saving Guide to Emulsifier Selection", publié en 1984 par ICI Americas, Inc., Wilmington, Delaware, Généralement, les agents émulsionnants pour les émulsions huile-dans-l'eau ont des indices HLB compris entre 6 et 18.

Dans la mise en oeuvre de la présente invention, il est souhaitable que l'agent émulsionnant présente un indice HLB compris entre 6 et 10, de préférence entre 7 et 9. Les modes de réalisation préférés de l'invention utilisent un mélange d'agents émulsionnants dont l'indice HLB est d'environ 8.

En plus de l'indice HLB, un agent émulsionnant peut être classé en fonction de sa classe chimique. Bien que des agents émulsionnants de différentes classes chimiques puissent être utilisés dans la mise en oeuvre de la présente invention, les alcools gras éthoxylés constituent la classe d'agents émulsionnants préférée, en particulier, ceux qui comportent des segments d'alcool gras ayant de 10 à 24 atomes de carbone. Les alcools gras éthoxylés (appellés également alcools polyoxyéthylènés ou éthers d'alcools gras de polyéthylène glycol) sont des tensioactifs non ioniques préparés par éthoxylation d'alcools gras avec l'oxyde d'éthylène. Il est possible de préparer une grande variété d'agents émulsionnants avec une gamme étendue de propriétés en faisant varier l'alcool gras utilisé (segment lipophile) et le degré de polymérisation du polyéthylène glycol (segment hydrophile). Les agents émulsionnants préférés comprennent une partie d'alcool cétylique, laurique, myristique, oléique, stéarylique ou tridécylique, et une partie polyéthylène glycol ayant un nombre moyen d'unités d'oxyde d'éthylène compris entre 2 et 25.

Un mélange de steareth-2 et de steareth-20, des alcools gras éthoxylés comprenant des parties alcool stéarylique et des parties de polyéthylène glycol avec respectivement 2 et 20 unités d'oxyde d'éthylène, est le mélange d'agents émulsionnants préféré.

La proportion d'agent émulsionnant est fonction du type d'agent émulsionnant utilisé, de la quantité de vaseline et de la quantité d'eau utilisées. Dans les modes de réalisation comprenant 20 % en poids de vaseline, 65-70 % en poids d'eau et un agent émulsionnant comprenant un mélange de steareth-2 et de steareth-20, la quantité d'agent émulsionnant est d'environ 2,5 % en poids du poids total de l'émulsion.

Bien que ce ne soit pas essentiel pour l'invention, il peut être souhaitable dans certains cas d'ajouter de faibles proportions d'amidon, afin d'améliorer encore l'élégance cosmétique et réduire l'effet collant de la composition finale de mousse. Les amidons appropriés sont ceux qui ne gonflent pas facilement en présence d'eau, et qui absorbent une quantité considérable d'eau sans former une pâte.

Les amidons hydrophobes sont particulièrement appropriés. Un amidon hydrophobe est un amidon qui a été modifié en y introduisant des groupes hydrophobes qui lui confèrent un caractère hydrophobe plutôt qu'hydrophile, comme le sont les amidons conventionnels non modifiés.

Les formes les plus courantes d'amidon hydrophobe sont les esters d'amidon contenant des groupes hydrophobes et des éthers complexes d'amidon. Un exemple spécifique d'un amidon hydrophobe approprié est un produit commercialisé sous le nom de DRY FLO®.

Le DRY FLO® est un sel d'aluminium d'un semi-ester d'amidon faiblement substitué de l'acide octényl succinique. Il est extrêmement résistant à la mouillabilité par l'eau, tout en conservant la capacité d'un amidon à absorber l'eau sans gonfler. Cet amidon est également caractérisé par ses propriétés d'écoulement libre, même après absorption d'une quantité important d'eau. Bien que la proportion d'amidon hydrophobe tel que le DRY FLO ne soit pas critique pour l'invention, une proportion inférieure à environ 10 % en poids et généralement d'environ 1 % à environ 10 %, et de préférence d'environ 3 % en poids de la quantité totale de l'émulsion huile-dans-l'eau s'est révélée appropriée.

Il peut être également souhaitable d'ajouter un silicone pour améliorer l'aptitude à l'étalement du produit final et améliorer encore son élégance cosmétique. Il est souhaitable que le silicone soit un silicone volatile, comme le cyclométhicone, et qu'il soit présent dans une proportion inférieure à 10 % en poids de l'émulsion totale, et généralement d'environ 1 % en poids à 10 % en poids.

Comme ceci est bien connu en cosmétique, de faibles proportions d'autres ingrédients peuvent également être ajoutées à l'émulsion pour conférer des caractéristiques souhaitables au produit final. Ces ingrédients sont par exemple, des agents conservateurs, des stabilisants, des neutralisants, des ajusteurs de viscosité, et des parfums.

D'une manière générale, le propulseur de l'invention est une substance organique volatile qui existe sous forme de gaz à la température ambiante, sous forme liquide sous des pressions élevées praticables dans des récipients appropriés, et présente une faible solubilité dans l'eau. Le propulseur doit être d'une nature telle qu'il ne détruise pas la mousse ou ne décompose pas l'émulsion produisant la mousse dans le récipient. Généralement, le propulseur présente une pression de vapeur dans le domaine d'environ 34,5 à environ 1380 x 10 Pa et de préférence d'environ 138 x 10 Pa à environ 483 x 10 Pa à 21 ° C. Le propulseur peut être un mélange de deux ou plusieurs composés qui a une pression de vapeur dans le domaine désiré, bien que les composés en eux-mêmes puissent présenter des pressions de vapeur qui sont en dehors du domaine souhaité. La solubilité dans l'eau du propulseur ou du mélange de propulseur doit être telle que le propulseur se trouve essentiellement sous forme de phase liquide non dissoute dans l'émulsion, lorsque les deux sont mélangés sous une pression suffisante pour maintenir le propulseur dans la phase liquide.

En particulier, lorsque la mousse finale est utilisée pour produire une composition de traitement de la peau, il peut être souhaitable d'éviter l'utilisation de propulseurs qui produisent une sensation de picotement ou de brûlure lorsque la composition est appliquée sur la peau. Les hydrocarbures aliphatiques saturés à chaîne droite, à chaîne ramifiée ou cycliques, ayant une pression de vapeur appropriée, tels que le propane, le butane, l'isobutane et le cyclobutane, sont appropriés comme propulseur produisant de la mousse et ne provoquent pas de sensation désagréable de brûlure. On peut également utiliser, en particulier en mélange avec d'autres propulseurs des hydrocarbures aliphatiques saturés, partiellement mais pas totalement fluorés, ayant une pression de vapeur appropriée, tels que le difluoroéthane.

La proportion de propulseur utilisée selon l'invention n'est pas critique mais elle détermine la densité de la mousse produite. Plus la proportion de propulseur est élevée, plus la densité de la mousse est faible. Généralement, les densités de mousse habituelles sont situées dans le domaine d'environ 0,02 à environ 0,20 g/cm3. Les densités de mousse souhaitables sont situées dans le domaine d'environ 0,05 à 0,15 g/cm3. Les densités dans ce domaine préféré peuvent être obtenues en utilisant de 5 % à environ 10 % en poids de propulseur par rapport au poids total du propulseur et de la composition d'émulsion. Le pourcentage en poids de propulseur nécessaire pour produire une densité de mousse donnée varie en fonction du poids moléculaire du propulseur utilisé. Lorsqu'on utilise un mélange de propulseurs comprenant 60 % en poids de difluoroéthane et 40 % en poids de butane ou d'isobutane, la proportion de

4

propulseur par rapport au poids de la composition est d'environ 5 % à environ 10 % pour des densités de mousse d'environ 0,05 à environ 0,15 g/cm3.

La composition est contenue de préférence dans un récipient tel que décrit ci-dessus, à partir duquel elle est propulsée à volonté par la pression du gaz propulseur dans le volume de tête du récipient.

Lorsque la quantité de la composition liquide dans le récipient, décroît, la concentration de propulseur dans a composition liquide baisse, en raison du fait qu'une partie du propulseur s'évapore pour remplir l'accroissement du volume de tête. Ainsi, la densité de la mousse augmente lorsque le contenu du récipient diminue. La densité d'une mousse produite par des aérosols connus peut varier dans une large mesure entre le moment où le récipient est plein et le moment où le récipient est pratiquement vide. Les variations typiques peuvent se monter jusqu'à 70 %, par exemple d'environ 0,07 g/cm3 comme densité de mousse initiale jusqu'à environ 0,12 g/cm3 comme densité de mousse finale.

Un autre aspect de la présente invention consiste à fournir un procédé de production d'une mousse à partir d'un diffuseur aérosol, qui comprend la diffusion d'une émulsion huile-dans-l'eau décrite ci-dessus avec un propulseur produisant ainsi une mousse ayant une densité de mousse pratiquement constante, depuis la diffusion initiale jusqu'à la diffusion finale. De préférence, la mousse présente une densité qui varie de moins de 30 % depuis la diffusion initiale jusqu'à la diffusion finale. Le procédé comprend la diffusion d'une émulsion huile-dans-l'eau avec un propulseur, dans des conditions permettant de produire une mousse, l'émulsion comprenant au moins 10 % en poids de vaseline, au moins 50 % en poids d'eau et un agent émulsionnant ayant une balance hydrophile-lipophile d'environ 6 à 10. La diffusion initiale signifie ici une diffusion lorsque le récipient contient environ 85 %-90 % en poids de la quantité totale d'émulsion introduite initialement dans ledit récipient. La diffusion finale signifie ici la diffusion lorsque le récipient contient environ 15 % en poids de la quantité totale de l'émulsion introduite initialement dans ledit récipient.

Le récipient de la présente invention est rempli avec les quantités d'émulsion et de propulseur souhaitées, soit séparément soit simultanément. Les composants du propulseur sont introduits de préférence sous une pression supérieure à leur pression de vapeur à la température donnée, de sorte que le propulseur est sous forme liquide, excepté la faible proportion sous forme gazeuse qui remplit le volume de tête. La composition est mélangée dans le récipient par agitation induite par l'addition des composants ou lorsque l'utilisateur secoue le récipient. En conséquence, l'émulsion doit présenter une viscosité qui lui permet de couler aux températures et pressions régnant dans le récipient. Il est souhaitable que l'émulsion présente une viscosité comprise entre environ 1 Pa.s et 15 Pa.s, de préférence environ 7 Pa.s.

L'invention sera illustrée à l'aide des exemples suivants. Ces exemples sont indiqués pour une meilleure compréhension de l'invention, mais ne doivent en aucune façon être considérés comme en limitant la portée.

EXEMPLE 1

| Concentrat d'émulsion d'aérosol | |
|---|---|
| Ingrédients | % en poids de l'émulsion totale |
| Eau | 68,55 |
| Vaseline | 20,00 |
| Cyclométhicone (silicone) | 5,00 |
| Amidon octénylsuccinate d'aluminium | 3,00 |
| Steareth-2 (émulsionnant) | 1,73 |
| Steareth-20 (émulsionnant) | 0,58 |
| Imidazolidinyl urée (conservateur) | 0,30 |
| Méthylparaben (conservateur) | 0,20 |
| Propylparaben (conservateur) | 0,15 |
| Carbomer 941 (stabilisant) (Acide polyacrylique réticulé avec un agent polyfonctionnel) | 0,12 |
| Triéthanolamine (agent neutralisant) | 0,12 |
| Mélange de cétyl-stéaryl sulfate de sodium (agent co-émulsionnant) | 0,10 |
| Alcool cétylique (ajusteur de viscosité) | 0,10 |
| EDTA disodique (stabilisant) | 0,05 |

## Composition d'aérosol produisant la mousse

| Ingrédients | | | % en poids |
|---|---|---|---|
| Concentrat d'émulsion d'aérosol | | | 93-95 % |
| Mélange de propulseurs | Difluoroéthane | 60 % | 5-7 % |
| | Butane | 40 % | |

EXEMPLE 2

| Concentrat d'émulsion d'aérosol | |
|---|---|
| Ingrédients | % en poids de l'émulsion totale |
| Eau | 68,55 |
| Vaseline | 20,00 |
| Cyclométhicone (silicone) | 5,00 |
| Amidon octénylsuccinate d'aluminium | 3,00 |
| Ceteth-2 (émulsionnant) | 1,70 |
| Ceteth-20 (émulsionnant) | 0,61 |
| Imidazolidinyl urée (conservateur) | 0,30 |
| Méthylparaben (conservateur) | 0,20 |
| Propylparaben (conservateur) | 0,15 |
| Carbomer 941 (stabilisant) | 0,12 |
| Triéthanolamine (agent neutralisant) | 0,12 |
| Mélange de cétyl-stéaryl sulfate de sodium (agent co-émulsionnant) | 0,10 |
| Alcool cétylique (ajusteur de viscosité) | 0,10 |
| EDTA disodique (stabilisant) | 0,05 |

## Composition d'aérosol produisant de la mousse

| Ingrédients | | | % en poids |
|---|---|---|---|
| Concentrat d'émulsion d'aérosol | | | 93-95% |
| Mélange de propulseurs | Difluoréthane | 60 % | 5-7% |
| | Butane | 40 % | |

EXEMPLE 3

Préparation du concentrat d'émulsion d'aérosol de l'exemple n° 1. Faire chauffer environ 90 % de l'eau à environ 70°C. Introduire le Carbomer 941 dans l'eau sous agitation modérée et poursuivre l'agitation jusqu'à dispersion complète (environ une heure). Tout en maintenant à 70 °C, ajouter le mélange de cétyl-stéaryl sulfate de sodium l'EDTA disodique, et le méthylparaben.

Combiner la vaseline, le steareth-2, le steareth-20, le propylparaben, et faire chauffer à 70°C sous agitation lente jusqu'à la fusion de cette phase.

Ajouter lentement à la phase aqueuse la phase huileuse ci-dessus, sous une agitation à force de cisaillement élevée (Homo-mixer). Après la formation de l'émulsion, ajouter la triéthanolamine.

Laisser refroidir le mélange à 50°C sous agitation continue dans le Homo-mixer et ensuite, ajouter l'imidazolidinyl urée et le reste de l'eau.

A 50°C ou en dessous, ajouter le cyclométhicone et ensuite, faire tomber en pluie l'amidon octénylsuccinate d'aluminium pendant que le mélange continue à se refroidir. Laisser refroidir le mélange à 30°C sous agitation lente.

EXEMPLES 4-7

| Concentrat d'émulsion d'aérosol | | | | |
|---|---|---|---|---|
| ingrédients | % en poids de l'émulsion totale | | | |
| | Ex.4 | Ex.5 | Ex.6 | Ex.7 |
| Eau | 76.05 | 71.05 | 63.55 | 58.55 |
| Vaseline | 12.50 | 17.50 | 25.00 | 30.00 |
| Cyclométhicone (silicone) | 5.00 | 5.00 | 5.00 | 5.00 |
| Amidon octénylsuccinate d'aluminium | 3.00 | 3.00 | 3.00 | 3.00 |
| Steareth-2 (émulsionnant) | 1.73 | 1.73 | 1.73 | 1.73 |
| Stéareth-20 (émulsionnant) | 0.58 | 0.58 | 0.58 | 0.58 |
| Imidazolidinyl urée (conservateur) | 0.30 | 0.30 | 0.30 | 0.30 |
| Méthylparaben (conservateur) | 0.20 | 0.20 | 0.20 | 0.20 |
| Propylparaben (conservateur) | 0.15 | 0.15 | 0.15 | 0.15 |
| Carbomer 941 (stabilisant) | 0.12 | 0.12 | 0.12 | 0.12 |
| Triéthanolamine (agent neutralisant) | 0.12 | 0.12 | 0.12 | 0.12 |
| Mélange de cétyl-stéaryl sulfate de sodium (agent co-émulsionnant) | 0.10 | 0.10 | 0.10 | 0.10 |
| Alcool cétylique (ajusteur de viscosité) | 0.10 | 0.10 | 0.10 | 0.10 |
| EDTA disodique (stabilisant) | 0.05 | 0.05 | 0.05 | 0.05 |

Compositions d'aérosol produisant

la mousse

A. Ingrédients       % en poids

Concentrat d'émulsion d'aérosol       95 %

     (Exemples 4 et 5)

Mélange de propulseurs { Difluoroethane 60 %      5 %
Butane 40 %.

B. Ingrédients       % en poids

   Concentrat d'émulsion d'aérosol       93 %

     (Exemples 6 et 7)

Mélange de propulseurs { Isobutane 80 %      7 %
Propane 20 %

**Revendications**

1.  Composition aérosol pour la formation d'une mousse, destinée à une application topique, comprenant une émulsion huile-dans-l'eau et un propulseur conditionnés sous pression dans un récipient aérosol, caractérisée par le fait que ladite émulsion huile-dans-l'eau comprend au moins 10 % en poids de vaseline, au moins 50 % en poids d'eau et un agent émulsionnant ayant une balance hydrophile-lipophile de 6 à 10, et par le fait que ladite mousse a une densité de mousse qui varie de moins de 30 % entre la diffusion initiale et la diffusion finale de la composition à partir du récipient aérosol.

2.  Composition selon la revendication 1, dans laquelle la dite émulsion huile-dans-l'eau comprend de 10 à 40 % en poids de vaseline, de 50 à 80 % en poids d'eau et de 2 à 10 % en poids d'un agent émulsionnant par rapport au poids total de la dite émulsion.

3.  Composition selon la revendication 1 ou 2, dans laquelle ladite émulsion huile-dans-l'eau contient également un amidon ou un ester d'amidon modifié hydrophobe de préférence l'amidon octénylsuccinate d'aluminium.

4.  Composition selon l'une des revendications 1 à 3, dans laquelle ladite émulsion huile-dans-l'eau contient également un silicone volatile de préférence le cyclométhicone

5.  Composition selon la revendication 1 ou 2 dans laquelle ledit agent émulsionnant présente une balance hydrophile-lipophile de 7 à 9 et de préférence d'environ 8.

6.  Composition selon la revendication 1 ou 2 dans laquelle ledit agent émulsionnant est un alcool gras éthoxylé ayant une partie de polyéthylène glycol contenant un nombre moyen d'unités d'oxyde d'éthylène compris entre 2 et 25.

7.  Composition selon la revendication 6 dans laquelle ledit alcool gras éthoxylé comprend une partie d'alcool cétylique, laurique, myristique, oléique, stéarylique ou tridécylique.

8.  Composition selon l'une quelconque des revendications précédentes, dans laquelle ledit agent émulsionnant est le steareth-2 ou le steareth-20 ou un mélange de ceux-ci.

9.  Composition selon l'une quelconque des revendications précédentes qui comprend également au moins un agent stabilisant, un agent neutralisant, un ajusteur de la viscosité, un conservateur ou un parfum.

10. Composition selon l'une quelconque des revendications précédentes, dans laquelle ladite émulsion huile-dans-l'eau comprend environ 20 % en poids de vaseline , environ 68 % en poids d'eau, environ 2,5 % en poids d'un mélange de steareth-2 et de steareth-20, environ 5 % en poids de cyclométhicone, et environ 3 % en poids d'un amidon octénylsuccinate d'aluminium.

11. Composition selon la revendication 1, dans laquelle ledit propulseur comprend au moins un hydrocarbure ou un hydrocarbure fluoré, ou un mélange de ceux-ci.

12. Composition selon la revendication 11, dans laquelle ledit propulseur comprend au moins du butane, de l'isobutane, du difluoréthane, ou un mélange de ceux-ci.

13. Composition selon la revendication 1, dont la mousse présente une densité d'environ 0,02 à 0,20 g/cm$^3$ et de préférence d'environ 0,05 à 0,15 g/cm$^3$.

**Claims**

1.  Aerosol composition for the formation of a foam, intended for topical application, comprising an oil-in-water emulsion and a propellant packaged under pressure in an aerosol can, characterized in that the said oil-in-water emulsion comprises at least 10 % by weight of vaseline, at least 50 % by weight of water and an emulsifying agent having a hydrophilic-lipophilic balance of 6 to 10, and in that the said foam has a foam density which varies by less than 30 % between the initial diffusion and the final diffusion of the composition from the aerosol can.

2. Composition according to Claim 1, in which the said oil-in-water emulsion comprises from 16 to 40 % by weight of vaseline, from 50 to 80 % by weight of water and from 2 to 10 % by weight of an emulsifying agent relative to the total weight of the said emulsion.

3. Composition according to Claim 1 or 2, in which the said oil-in-water emulsion also contains a starch or a hydrophobic modified starch ester, preferably aluminium starch octenylsuccinate.

4. Composition according to one of Claims 1 to 3, in which the said oil-in-water emulsion also contains a volatile silicone, preferably cyclomethicone.

5. Composition according to Claim 1 or 2, in which the said emulsifying agent possesses a hydrophilic-lipophilic balance of 7 to 9, and preferably approximately 8.

6. Composition according to Claim 1 or 2, in which the said emulsifying agent is an ethoxylated fatty alcohol having a polyethylene glycol portion containing an average number of ethylene oxide units of between 2 and 25.

7. Composition according to Claim 6, in which the said ethoxylated fatty alcohol comprises a cetyl, lauryl, myristyl, oleyl, stearyl or tridecyl alcohol portion.

8. Composition according to any one of the preceding claims, in which the said emulsifying agent is steareth-2 or steareth-20 or a mixture of these.

9. Composition according to any one of the preceding claims, which also comprises at least one stabilizing agent, neutralizing agent, viscosity adjuster, preservative or fragrance.

10. Composition according to any one of the preceding claims, in which the said oil-in-water emulsion comprises approximately 20 % by weight of vaseline, approximately 68 % by weight of water, approximately 2.5 % by weight of a mixture of steareth-2 and steareth-20, approximately 5 % by weight of cyolomethicone and approximately 3 % by weight of an aluminium starch octenylsuccinate.

11. Composition according to Claim 1, in which the said propellant comprises at least one hydrocarbon or fluorinated hydrocarbon, or a mixture of these.

12. Composition according to Claim 1, in which the said propellant comprises at least butane, isobutane, difluoroethane or a mixture of these.

13. Composition according to Claim 1, in which the foam possesses a density of approximately 0.02 to 0.20 g/cm$^3$, and preferably approximately 0.05 to 0.15 g/cm$^3$.

**Patentansprüche**

1. Aerosolschaummittel zur Anwendung auf der Haut, das eine Öl-in-Wasser-Emulsion und ein Treibmittel umfaßt, die in einem Aerosol-Behälter unter Druck konditioniert sind,
**dadurch gekennzeichnet,** daß
die Öl-in-Wasser-Emulsion mindestens 10 Gew.-% Vaseline, mindestens 50 Gew.-% Wasser und ein Emulgiermittel mit einem Hydrophil-Lipophil-Gleichgewicht von 6 bis 10 umfaßt, und daß der Schaum eine Schaumdichte aufweist, die um weniger als 30 % zwischen der ersten Abgabe und der letzten Abgabe des Mittels aus dem Aerosolbehälter variiert.

2. Mittel nach Anspruch 1, worin
die Öl-in-Wasser-Emulsion 10 bis 40 Gew.-% Vaseline, 50 bis 80 Gew.-% Wasser und 2 bis 10 Gew-% eines Emulgiermittels, bezogen auf das Gesamtgewicht der Emulsion, umfaßt.

3. Mittel nach Anspruch 1 oder 2, worin
die Öl-in-Wasser-Emulsion ebenfalls Stärke oder einen modifizierten hydrophoben Stärkeester, vorzugsweise Aluminiumoctenylsuccinatstärke, enthält.

4. Mittel nach einem der Ansprüche 1 bis 3, worin
die Öl-in-Wasser-Emulsion ebenfalls ein flüchtiges Silikon, vorzugsweise Cyclomethikon, enthält.

5. Mittel nach einem der Ansprüche 1 oder 2, worin
das Emulgiermittel ein Hydrophil-Lipophil-Gleichgewicht von 7 bis 9, vorzugsweise von etwa 8, aufweist.

6. Mittel nach Anspruch 1 oder 2, worin
das Emulgiermittel ein ethoxylierter Fettalkohol mit einem Polyethylenglykolanteil ist, der eine mittlere Anzahl von Ethylenoxideinneiten von 2 bis 25 aufweist.

7. Mittel nach Anspruch 6, worin
der ethoxylierte Fettalkohol einen Cetyl-, Lauryl-, Myristyl-, Oleyl-, Stearyl- oder Tridecylalkoholrest aufweist.

8. Mittel nach einem der Vorhergehenden Ansrpüche, worin
das Emulgiermittel Steareth-2 oder Steareth-20 oder ein Gemisch davon ist.

9. Mittel nach einem der vorhergehenden Ansprüche, das ebenfalls mindestens ein Stabilisierungsmittel, ein Neutralisierungsmittel, ein Mittel zur Einstellung der Viskosität, ein Konservierungsmittel oder ein Parfüm enthält.

10. Mittel nach einem der vorhergehenden Ansprüche, wobei
die Öl-in-Wasser-Emulsion etwa 20 Gew.-% Vaseline, etwa 68 Gew.-% Wasser, etwa 2,5 Gew.-% eines Gemisches aus Steareth-2 und Steareth-20, etwa 5 Gew.-% Cyclomethikon und etwa 3 Gew.-% einer Aluminiumoctenylsuccinatstärke umfaßt.

11. Mittel nach Anspruch 1, wobei
das Treibmittel mindestens einen Kohlenwasserstoff oder fluorierten Kohlenwasserstoff oder ein Gemisch davon umfaßt.

12. Mittel nach Anspruch 1, wobei
das Treibmittel mindestens Butan, Isobutan, Difluorethan oder ein Gemisch davon umfaßt.

13. Mittel nach Anspruch 1, wobei
der Schaum eine Dichte von etwa 0,02 bis 0,20 g/cm$^3$ und vorzugsweise von etwa 0,05 bis 0,15 g/cm$^3$ aufweist.